# EUROPEAN PATENT SPECIFICATION

(11) **EP 0 428 512 B1**
(45) Date of publication and mention of the grant of the patent: **17.11.1994**
(21) Application number: 89901928.5
(22) Date of filing: 27.12.1988
(51) Int. Cl.: C01B 13/11

(54) **OZONE GENERATION BY CORONA DISCHARGE AT ELEVATED PRESSURES**
OZONHERSTELLUNG DURCH KORONAENTLADUNG BEI ERHÖHTEM DRUCK
PRODUCTION D'OZONE PAR DECHARGE LUMINEUSE A DES PRESSIONS ELEVEES

(43) Date of publication of application: 29.05.1991
(73) Proprietor: Karlson, Eskil Leannant, Erie Pennsylvania 16509 (US)
(72) Inventor: Karlson, Eskil Leannant, Erie Pennsylvania 16509 (US)
(74) Representative: Keil, Rainer A., Dipl.-Phys. Dr.
(86) International application number: US8804687
(87) International publication number: WO9007466

(56) References cited:
- DE-A- 3 324 939
- US-A- 2 823 076
- US-A- 2 899 281
- US-A- 2 936 279
- US-A- 3 153 577
- US-A- 3 170 633
- US-A- 3 225 972
- US-A- 3 464 797
- US-A- 3 704 096
- US-A- 3 899 685
- US-A- 4 025 441
- US-A- 4 079 260
- US-A- 4 119 486
- US-A- 4 214 905
- US-A- 4 229 252
- US-A- 4 234 800
- US-A- 4 240 798
- US-A- 4 409 183
- US-A- 4 614 573
- US-A- 4 640 782
- US-A-39 636 002
- Chemical Abstract, vol. 84, no. 12, issued 1976, 29 March (Columbus, Ohio, USA), E.L. Karlson (Life Support Syst. Inc., Stamford Conn. USA). "Use of Ozone in Pollution Control" see page 353, column 1, abstract no. 79206n, Pollut. Eng. Tech., Pap. Int. Pollut Engr. Congr., 3rd 1974, 139-43 (Eng).
- Chemical Abstract, vol. 101, no. 24, issued 1984 10 December, (Columbus Ohio USA), E. Karlson (Life Support Syst., Erie, PA, USA); "Karlson Ozone Sterilizer. Final Report." see page 313, column 2, abstract no. 2163816, Report 1984, DOE/CE/25082-T1; order no. DE84011489, 110pp (Eng).

## Description

This invention is an ozone generation system that teaches the method to employ very high pressure within the generator to produce ozone using less energy, up to 50% less dependent on pressure and temperature, than a system operating at ambient temperature and pressure.

A representative of the prior art is US-A-2 936 279 disclosing an apparatus for ozonizing oxygen containing gases under pressure.

In drawing, Fig. 1 is a basic diagram of a corona discharge ozone generating system using air or oxygen at atmospheric pressure. Fig. 2 is a basic diagram of a basic corona discharge ozone generating system using oxygen at pressures of 20.6·10⁶ N/m² (3,000 pounds per square inch). Fig. 3 is a sectional elevation of an ozone generator using oxygen at pressures of 3,000 20.6·10⁶ N/m² (pounds per square inch), and Fig. 4 is a section through a modification of the high pressure ozone generator, Fig. 5 is a diagram of the ozone generating electronic pulses.

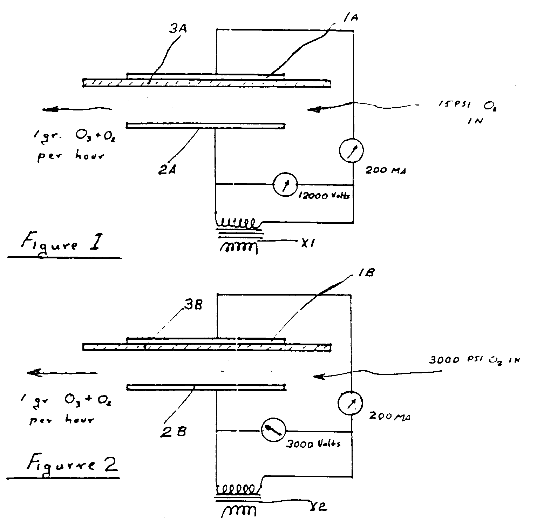

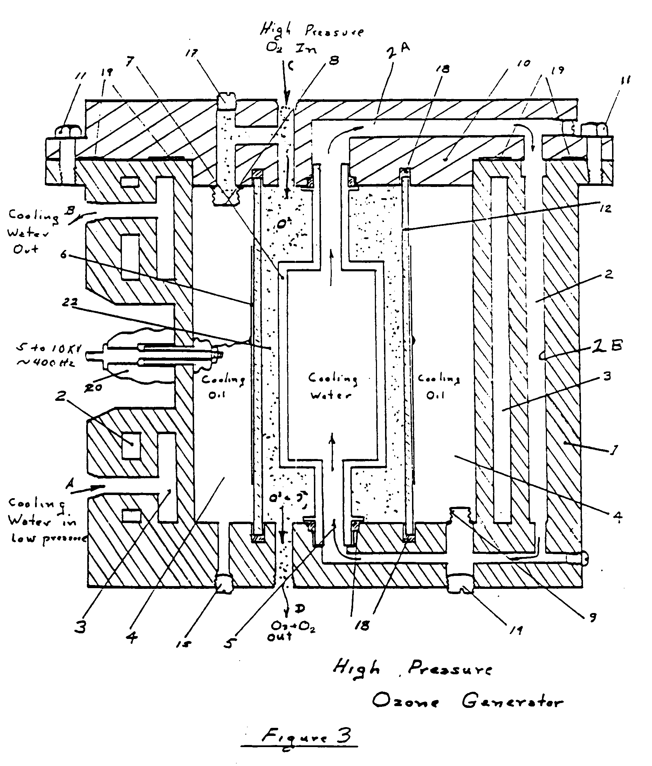

In the corona discharge ozone generating system of Fig. 1 is a diagram of a high alternating voltage, for example 10 Kv at 400 Hz, the transformer is connected across a metal electrode 1a and a metal electrode 2a plated on a dielectric such as glass 3a. Oxygen at atmospheric pressure flows through the space between the glass and the electrodes 1a and 2a. The voltage is sufficiently high to cause ionization of the oxygen but is below the value at which, break down and sparing occurs. The power consumed in the ozone generation is indicated by an ammeter 4a. Fig. 2 shows the same apparatus of Fig. 1 supplied with oxygen under a pressure of 20.6·10⁶ N/m² (3,000 pounds per square inch). By lowering the voltage applied across the electrodes 1b, 2b until the current indicated by ammeter 4b is the same as previously noted 4a when the apparatus was supplied with oxygen at atmospheric pressure, the ozone output is greater than the output from the atmospheric pressure oxygen.

The lower voltage indicates that the power required for ozone generation is decreased by the increased operating pressure. This has been tested for oxygen pressures of 3.4·10⁶ N/m² (500 pounds)to 20.6·10⁶ N/m² (3,000 pounds per square inch). It was found the higher the pressure the greater the yield of ozone. There is no reason why the same results should not be obtained with oxygen pressures above 20.6·10⁶ N/m² (3,000 pounds per inch).

The advantage of having a high pressure oxygen in the corona area, where the ozone is being produced, is that without changing any other parameters more ozone is produced per watt hour. At high pressure, the flow rate of oxygen must be increased, otherwise, the corona will act to destroy the ozone it generated. The temperature of the glass insulator also increases when the gas pressure is increased. The reason for this temperature increase is that more energy has passed through the glass and resistance across the high pressure gas area is lower. The advantage is that the glass insulator dielectric has not changed and its ac resistance remains nearly the same. In practice the high voltage was lowered to the same current level as when operating at low gas pressure, and the system used less energy in watts, but produced more ozone.

Fig. 3 is a center cut slice view of the internal structure of the high pressure ozone generator. This generator is made up of four major members, container 1, cover 10, glass or ceramic insulator 12, and inner metal electrode 7.

The outside container 1 is made up of three cooling compartments 2, 3, 4. Compartments 2 and 4 are hermetically sealed and filled with incompressible liquids. Compartment 2 is filled with water which cools the center or ground electrode 7 by conduction and convection - the hot water leaves the center electrode by rising up through the end plate 10, down through cannel 2b and after being cooled, it rises back going up through 5 into the center electrode. For increased cooling a sealed pump can be added in line 2b.

The second cooling compartment 3 extracts the heat from both the sealed internal cooling water of compartment 2 and the sealed inner oil of compartment 4. The inner oil compartment 4 cools and insulates the glass tube 12 which is plated with silver over a portion of its external surface to provide high voltage electrode 6 next to the oil.

The cooling water for chamber 3 enters at point A, exits at point B. This cooling chamber lies between the oil cooling and the water cooling chambers 4 and 2. This cooling water, cools both the oil which controls the temperature of the glass insulation tube 12 and the water which controls the temperature of the center ground electrode 7.

The container part 1 also has welded to its bottom a pressure equalizer bellows 9. This equalizer sets the water pressure in the ground electrode cooling loop 2 at the same pressure as the oil pressure in the oil cooling chamber 4 for the high voltage electrode 6.

The second part 10, the cover, is held to part 1 by a ring of screws 11 around its edge as is shown in Fig. 3. The two high pressure metal rings of packing 19 seal the top 10 to the container 1. The cover 10 also aligns both the glass insulator 12 and the center electrode 7.

The cover end plate 10 also has attached to it, projecting into the oil 4, down from cover 10 a pressure equalizer bellows 8 which transmits the pressure of the high pressure oxygen to the oil. The pressure equalizers 8 and 9 are each made of a bellows of thin nickel welded to the top plate 10 and the bottom of the tank 1. As the pressure is increased, the oxygen builds upcoming in at point C (in the cover 10) both 8 and 9 will equalize the pressure without mixing of water, oxygen and oil. The pressure across the glass insulator will never be more than one pound no matter what the input oxygen pressure at point C is. In all cases both the water and oil hermetically sealed cooling systems must be full with little or no air in either system.

The third part 12, the glass insulator, is mounted in grooves in both the top cover 10 and the bottom of the container 1. Sealing is accomplished by a neoprene rubber ring coated with floura carbon grease 18 at either end of the tube as shown in Fig. 3. This seal must be able to stand ozone at concentrations up to 12%. It is important to note that by using the pressure equalizer less leak problems will occur since there is very little pressure across any of the seals except at 19. The glass tube 12 has a coating of silver plate 6 over its outer sides except for a clear area of two inches at either end. The silver is attached to a wire that leads to the center conductor of insulator 20. The high voltage source is attached to this point. The case 1 is grounded. The inner metal electrode 7, the fourth member, is mounted between the top plate 10 and the bottom of container 1 as shown in Fig. 3. "0" rings 18 seal both ends.

Generating the ozone at high pressures is more efficient - produces more grams of ozone per watt. In US-A-3,719,017, ozone and air or oxygen mixture were compressed for spraying at pressures of up to 6.88·10^{⁶} N/m² (1,000 pounds per square inch). It has been learned ozone can be used more efficiently at high pressures only if it is generated at high pressures. The heat of compression obtained when pumping an ozone gas mixture to a high pressure will destroy nearly all the ozone to a gas not usable for sterilization. Another advantage of producing ozone at high pressure is that when you release it to a lower pressure as it will get colder and will be active longer.

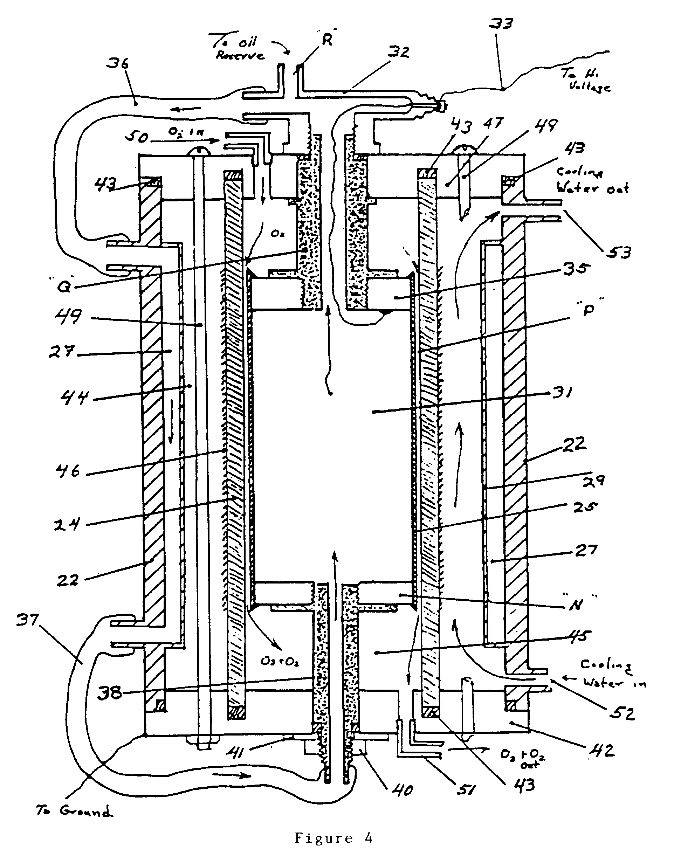

The ozone generator design shown in Fig. 4 has a number of parts: The metal, outer shell 22 encases the ozonator and carries on its inner surface a heat exchanger 27, 29 to cool the oil 31 which cools the inner electrode 25 and through the electrode cools the oxygen and ozone as they pass between the inner electrode and the glass insulator 24.

The oil is cooled by convection up through the inner electrode and down through heat exchanger 27. A pump could be attached in line at 37 to increase the flow rate and increase the cooling of oil from 31.

The plastic molding 32 at the top of the ozone generator serves several purposes: It supports the upper end of the inner insulator "Q" which is attached to 35 the metal ring holding 25 in place. Plastic element 32 also provides a passage for the flow by thermal convection of the heated oil out of the inner electrode to a high pressure plastic tube 36 on the way to the heat exchanger back through tube 37 and on to plastic molding 38, which returns the oil to the inside of inner electrode 25. Parts 32, "Q" and 38 are all produced of high strength filled teflon. Nut 40 over washer 41 holds teflon tube 38 in place in lower end plate 42.

Two 0-rings 43 are used at each end of the ozone generator to provide a seal between the cooling water space 44 and the gas space 45, support and protect the glass insulator 24 which has a conductive coating 46 on its outer surface to serve as the outer electrode. Two more 0-rings 43 seal the, outer shell 22 to the top plate 47 and the bottom plate 42 to prevent leakage of the cooling water 44. The complete unit is held together by a ring of six through-bolts 49, which also provide pressure on 0-rings 43 to assure leak-proof seals.

In operation, air or oxygen is pumped into the unit through inlet fitting 50 and then passes through a corona discharge between inner electrode 25 and insulator 24 where ozone is formed in "P" area. The resulting oxygen/ozone mixture is discharged from the unit through fitting 51 at the bottom of the unit. The cooling water enters at point 52 and exits at 53.

The advantage of Fig. 4 ozone generator is that the outside 22 is at ground potential and glass insulator 24 is water cooled and its outer surface next to the cooling water is also at ground potential. The use of water as a cooling agent for the glass is more effective than oil. The ozone generator in Fig. 4 can also be built to operate at high pressures by using the same method as was used in Fig. 3. It would only be necessary to design the full system to stand 20.6·10⁶ N/m² (3,000 pounds) and inserting an equalizing bellows in part "N" in the inner electrode in Fig. 4 and also adding an equalizing bellows in the wall at 29 between the oil and cooling water. The cooling water for a high pressure system would be a closed system having the water pumped from 53 in Fig. 4 to a heat exchanger (not shown) back to 52. No air should be left in the water cooling system. This cooling system must be designed to stand the same pressure as the oxygen that is fed to the ozone generator. The water and oil pressure is now equalized with the rest of the system as for Fig. 4 ozone generator.

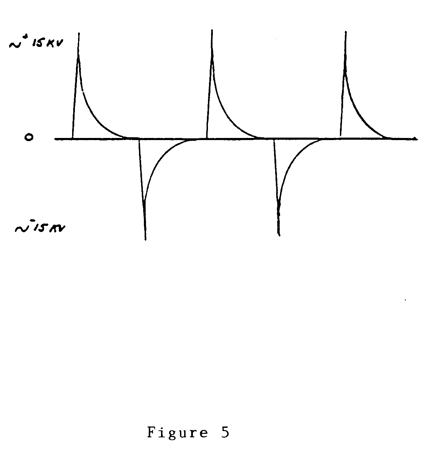

The ozone generator shown in Fig. 4 is an electrostatic device. This generator is driven by a step-up transformer (output over 23 kv) which in turn is excited by a dc supply - 120-160 V - which is chopped at a frequency in the range of 400 to 1500 Hz to produce a train of constant peak voltage spikes as shown in Fig. 5. The higher the spike frequency, the greater the ozone output will be.

There are a number of advantages to this new ozone generator. As shown in Fig. 4 the outer, metal shell 22 of the generator is at ground potential. The electrostatic driving system is so designed that only the 120-160 V dc supply and the high-voltage end of the step-up transformer are above ground.

The driving oscillator and the control circuits are all no more than 25 volts above ground. This system is transformer coupled to the Hi Power SCR's that chop the 120-160 Volts DC which drives the high voltage transformer. This ozone generator is water-cooled, with the water contacting the glass insulator 24 directly. The inner, stainless steel electrode 25 is oil-cooled by the oil-to-water heat exchanger 27 can the inner surface of the outer shell 22 to cool the oil. For more efficient cooling of the oil from the inner electrode 25 fins can be placed on the inner side of the 29.

Another advantage of our design is that a higher operating pressure may be used, which will produce more ozone per unit of applied energy. Since the cooling water is surrounding the glass insulator the oxygen can be supplied at a high pressure, the insulator 24 must then be loaded in compression by the cooling water at a pressure just slightly higher than oxygen pressure employed. The water pressure will counteract the pressure of the gas inside the glass insulator. The insulator will only have across it the differential pressure of the water and oxygen. An oxygen pressure of 20 atmospheres or higher can be employed as long as the cooling water is maintained at the same pressure or slightly higher. The pressure difference between the oil at 31 and the oxygen when operating with high pressure oxygen in the 45 area can be solved by adding a pressure equalizer at point "N" between the oil and the high pressure gas for the Fig. 4 ozone generator as shown in Fig. 3 at point 8 and 9. When using this technique no air can be present in the cooling oil circuit. For high pressure operation the oil and cooling water systems must be built so as not to move on a pressure change.

## Claims

1. An ozone generator provided with first and second electrodes (6, 7) spaced from each other, means for establishing a corona discharge from the first electrode to the second electrode and means for flowing oxygen or an oxygen containing gas at pressure higher than atmospheric pressure through said corona discharge area, characterized by a first hermetically sealed chamber (2) filled with a first cooling liquid under pressure and heat exchange relation to said first electrode (7), a second hermetically sealed chamber (4) filled with a second cooling liquid under pressure and heat exchange relation to said second electrode (6), flexible barrier means (8, 9) for equalizing or balancing pressure of the cooling liquids and the input gas, wherein the input gas is supplied under a pressure of about 3,44 x 10⁶ N/m² (500 psi) to about 6.89 x 10⁷ N/m² (10000 psi) to said corona discharge area.

2. The generator of claim 1, characterized in that the second electrode (6) is on an insulator (12).

3. The generator of claim 1 or 2, characterized in that it comprises catalyst platinum or iron oxide on the electrodes (6, 7) to increase the generation of ozone per unit of energy.

4. The generator of one or several of claims 1-3, characterized in that it comprises a tube of insulator material for the passage of the oxygen containing gas.

5. The generator of one or several of claims 1-4, characterized in that the first and second electrodes (6, 7) are concentric tubes being radially spaced from each other to provide a passageway through which the oxygen or oxygen containing gas flows.

6. The generator of one or several of claims 1-5, characterized in that a third cooling chamber (3) is provided between said first and second chamber (2, 4), a third fluid flowing through said third cooling chamber (3) and heat exchange relation to said first and second chamber (2, 4).

7. The generator of one or several of claims 1-6, characterized in that the hermetically sealed chambers (2, 4) are closed loops having sections of the loops in heat exchange relation to the cooling means (3).

8. The generator of one or several of claims 1-7, characterized by having means for applying the pressure of said gas to the liquid of at least one of said loops.

9. The generator of one or several of claims 1-8, characterized in that the second electrode (6) is on the outer surface of a insulator tube (12) with opposite ends sealed to the ends or headers (10) of a pressure vessel (1), an inlet (C) for oxygen or oxygen containing gas under pressure at one end of the tube (12) and an outlet (D) for ozone at the other end of the tube (12), the insulator tube (12) being subject to bursting pressure of the gas, and annular wall spaced outward from the insulator tube (12) and sealed to said headers providing a chamber (4) for a cooling fluid for said glass tube, and a bellows (8) sealed to said chamber for transmitting pressure from said gas to said cooling fluid for counterbalancing the bursting pressure on the glass tube.

10. The generator of one or several of claims 1-8, characterized in that the first and second electrodes (6, 7) are concentric tubes where the inner tube is metal and the outer tube is an insulator that is coated on its outside with a conductor, this conductor being the second electrode (6) and the high voltage electrode, and the inner metal tube being the first electrode (7) and at ground.

## Patentansprüche

1. Ozongenerator mit ersten und zweiten voneinander beabstandeten Elektroden (6, 7), Mitteln zur Erreichung einer Koronaentladung von der ersten Elektrode zu der zweiten Elektrode und Mitteln für den Fluß von Sauerstoff oder einen Sauerstoff enthaltenden Gas bei einem Druck, der größer ist als der Atmosphärendruck, durch den Koronaentladungsbereich, **gekennzeichnet durch** eine erste hermetisch abgedichtete Kammer (2), die mit einer ersten unter Druck stehenden Kühlflüssigkeit gefüllt ist und in Wärmeaustauschverbindung mit der ersten Elektrode (7) steht, eine zweite hermetisch abgedichtete Kammer (4), die mit einer zweiten unter Druck stehenden Kühlflüssigkeit gefüllt ist und in Wärmeaustauschverbindung mit der zweiten Elektrode (6) steht, flexible Begrenzungsmittel (8, 9) für das Ausgleichen oder Ausbalancieren von Druck der Kühlflüssigkeiten und des Eingangsgases, wobei das Eingangsgas unter einem Druck von etwa 3,44 x 10⁶ N/m² (500 psi) bis etwa 6,89 x 10⁷ N/m² (10000 psi) der Koronaentladungsfläche zugeführt wird.

2. Generator nach Anspruch 1, **dadurch gekennzeichnet**, daß die zweite Elektrode (6) auf einem Isolator (12) angeordnet ist.

3. Generator nach Anspruch 1 oder 2, **dadurch gekennzeichnet**, daß er Katalysatorplatin oder -eisenoxid auf den Elektroden (6, 7) aufweist, um die Herstellung von Ozon pro Energieeinheit zu erhöhen.

4. Generator nach einem oder mehreren der Ansprüche 1 bis 3, **dadurch gekennzeichnet**, daß er ein Rohr aus Isoliermaterial für den Durchgang des Sauerstoff enthaltenden Gases aufweist.

5. Generator nach einem oder mehreren der Ansprüche 1 bis 4, **dadurch gekennzeichnet**, daß die ersten und zweiten Elektroden (6, 7) konzentrische Rohre sind, die radial voneinander beabstandet sind, um einen Durchgang zu schaffen, durch den der Sauerstoff oder das Sauerstoff enthaltende Gas fließt.

6. Generator nach einem oder mehreren der Ansprüche 1 bis 5, **dadurch gekennzeichnet**, daß eine dritte Kühlkammer (3) zwischen der ersten und der zweiten Kammer (2, 4) vorgesehen ist, wobei ein drittes Fluid durch die dritte Kühlkammer (3) fließt und in Wärmeaustauschverbindung mit den ersten und zweiten Kammern (2, 4) steht.

7. Generator nach einem oder mehreren der Ansprüche 1 bis 6, **dadurch gekennzeichnet**, daß die hermetisch abgedichteten Kammern (2, 4) geschlossene Schleifen sind, wobei Abschnitte der Schleifen in Wärmeaustauschverbindung mit den Kühlmitteln (3) stehen.

8. Generator nach einem oder mehreren der Ansprüche 1 bis 7, **dadurch gekennzeichnet**, daß er Mittel zum Aufbringen des Druckes des Gases auf die Flüssigkeit wenigstens einer der Schleifen aufweist.

9. Generator nach einem oder mehreren der Ansprüche 1 bis 8, **dadurch gekennzeichnet**, daß die zweite Elektrode (6) auf der äußeren Fläche des Isolierrohres (12) angeordnet ist, wobei gegenüberliegende Enden mit den Enden oder Kopfabschnitten (10) eines Druckkessels (1) versiegelt sind, und durch einen Einlaß (C) für Sauerstoff oder Sauerstoff enthaltendes Gas unter Druck an einem Ende des Rohres (12) und einen Auslaß (D) für Ozon an dem anderen Ende des Rohres (12) liegt, wobei das Isolierrohr (12) dem Berstdruck des Gases ausgesetzt ist, und eine nach außen von dem Isolierrohr (12) beabstandete und mit den Kopfabschnitten versiegelte ringförmige Wand ist, wodurch eine Kammer (4) für eine Kühlflüssigkeit für das Glasrohr geschaffen wird, und durch einen Balg (8), der mit der Kammer für die Übertragung von Druck von dem Gas auf die Kühlflüssigkeit versiegelt ist, um den Berstdruck an dem Glasrohr auszugleichen.

10. Generator nach einem oder mehreren der Ansprüche 1 bis 8, **dadurch gekennzeichnet**, daß die ersten und zweiten Elektroden (6, 7) konzentrische Rohre sind, wobei das innere rohr aus Metall besteht und das äußere Rohr ein auf seiner Außenseite mit einem Leiter beschichteter Isolator ist, wobei der Leiter die zweite Elektrode (6) und die Hochspannungselektrode ist, und wobei das innere Metallrohr die erste Elektrode (7) und geerdet ist.

## Revendications

1. Générateur d'ozone muni de première et seconde électrodes (6, 7) espacées l'une de l'autre, un moyen pour établir une décharge par effet couronne depuis la première électrode jusqu'à la seconde électrode et un moyen pour faire circuler de l'oxygène ou un gaz contenant de l'oxygène à une pression plus élevée que la pression atmosphérique à travers ladite zone de décharge par effet couronne, caractérisé par une première chambre (2) fermée hermétiquement, remplie avec un premier liquide de refroidissement sous pression et dans une disposition d'échange mutuel de chaleur vis-à-vis de ladite première électrode (7), une seconde chambre (4) fermée hermétiquement, remplie avec un second liquide de refroidissement sous pression et dans une disposition d'échange mutuel de chaleur vis-à-vis de ladite seconde électrode (6), un moyen flexible formant barrière (8, 9) pour égaliser ou équilibrer la pression des liquides de refroidissement et du gaz d'entrée, le gaz d'entrée étant amené par la zone de décharge par effet couronne sous une pression comprise entre environ 3,44 X 10⁶ N/m² (500 psi) et environ 6,89 X 10⁷ N/m² (10000 psi).

2. Générateur selon la revendication 1, caractérisé en ce que la seconde électrode (6) se trouve sur un isolateur (12).

3. Générateur selon la revendication 1 ou 2, caractérisé en ce qu'elle comprend du platine ou de l'oxyde de fer en tant que catalyseur sur les électrodes (6, 7) pour augmenter la création d'ozone par unité d'énergie.

4. Générateur selon une ou plusieurs des revendications 1-3, caractérisé en ce qu'il comprend un tube en matière isolante pour le passage du gaz contenant de l'oxygène.

5. Générateur selon une ou plusieurs des revendications 1-4, caractérisé en ce que les première et seconde électrodes (6, 7) sont des tubes concentriques espacés radialement l'un de l'autre de manière à former un passage à travers lequel l'oxygène ou le gaz contenant de l'oxygène s'écoule.

6. Générateur selon une ou plusieurs des revendications 1-5, caractérisé en ce qu'une troisième chambre de refroidissement (3) est formée entre lesdites première et seconde chambres (2, 4), un troisième fluide s'écoulant à travers ladite troisième chambre de refroidissement (3) se trouvant dans une disposition d'échange mutuel de chaleur vis-à-vis des première et seconde chambres (2, 4).

7. Générateur selon une ou plusieurs des revendications 1-6, caractérisé en ce que les chambres fermées hermétiquement (2, 4) sont des boucles fermées dont les sections des boucles se trouvent dans une disposition d'échange mutuel de chaleur vis-à-vis du moyen de refroidissement (3).

8. Générateur selon une ou plusieurs des revendications 1-7, caractérisé par le fait qu'il comporte un moyen pour appliquer la pression dudit gaz au liquide d'au moins une desdites boucles.

9. Générateur selon une ou plusieurs des revendications 1-8, caractérisé en ce que la seconde électrode (6) se trouve sur la sur face extérieure d'un tube isolant (12) dont les extrémités opposées sont fixées de façon étanche aux extrémités ou collecteurs (10) d'un récipient de pression (1), une entrée (C) pour l'oxygène ou le gaz contenant de l'oxygène sous pression à une des extrémités du tube (12) et une sortie (D) pour l'ozone à l'autre extrémité du tube (12), le tube isolant (12) étant soumis à une pression d'éclatement du gaz, une paroi annulaire espacée du tube isolant (12) vers l'extérieur et fixée de façon étanche auxdits collecteurs formant une chambre (4) pour un fluide de refroidissement destiné au tube en verre précité, un soufflet (8) fixé de façon étanche à ladite chambre pour transmettre la pression dudit gaz audit fluide de refroidissement afin de contrecarrer la pression d'éclatement du tube de verre.

10. Générateur selon une ou plusieurs des revendications 1-8, caractérisé en ce que les première et seconde électrodes (6, 7) sont des tubes concentriques où le tube intérieur est en métal et le tube extérieur est en un matériau isolant qui est revêtu sur son côté extérieur d'un matériau conducteur, ce matériau conducteur constituant la seconde électrode (6) et l'électrode haute tension, et le tube métallique intérieur constituant la première électrode (7) et étant à la terre.
